# EUROPEAN PATENT APPLICATION

(11) **EP 4 825 426 A1**
(43) Date of publication of application: **21.10.2026**
(21) Application number: 25175732.4
(22) Date of filing: 12.05.2025
(51) Int. Cl.: A61M 25/00, A61M 25/10, A61M 29/02, A61B 17/22

(54) **EXPANSION DEVICE FOR EXPANDING TUBE IN HUMAN BODY**

(30) Priority: 18.04.2025 CN 202510496029; 18.04.2025 CN 202520755110 U
(71) Applicant: Hangzhou Hongyuan Medical Technology Co., Ltd., Hangzhou Zhejiang Province 311121 (CN)
(72) Inventor: Guo, Yinglu, Hangzhou, Zhejiang Province, 311121 (CN); Xing, Nianzeng, Hangzhou, Zhejiang Province, 311121 (CN); Xue, Wei, Hangzhou, Zhejiang Province, 311121 (CN); Ma, Zhifang, Hangzhou, Zhejiang Province, 311121 (CN); Gu, Baojun, Hangzhou, Zhejiang Province, 311121 (CN); Wen, Jiaming, Hangzhou, Zhejiang Province, 311121 (CN); Liu, Liwei, Hangzhou, Zhejiang Province, 311121 (CN); Liu, Jiasheng, Hangzhou, Zhejiang Province, 311121 (CN); Wang, Hao, Hangzhou, Zhejiang Province, 311121 (CN)
(74) Representative: Petraz, Gilberto Luigi

(57) **Abstract**

The invention provides an expansion device, including: a catheter body, configured to be capable of insertion into two organs in a human body or a mammal which are in communication with each other through a tube, and having a guide portion, an expansion portion and an infusion portion. The expansion portion includes an expansion balloon, and the expansion balloon is configured to be capable of expanding the tube between the two organs. The expansion device further includes a limiting element, and the limiting element is capable of limiting or controlling a size of expansion of the expansion balloon to keep the expansion balloon within a limited size. This technical solution can reduce a possibility of bursting of the expansion balloon under high pressure, and make the size of expansion of the tube consistent, thereby increasing the accuracy and precision of operation.

## Description

### CROSS-REFERENCE TO RELATED APPLICATIONS

This application claims priority to Chinese Patent Application, Application No.: 2025104960291,filed on April 18, 2025, 2025207551102,filed on April 18, 2025 ; and the specification, claims, abstract, and accompanying drawings of this application are incorporated by reference in their entirety as a part of this application.

### BACKGROUND OF THE INVENTION

### Field of the Invention

The disclosure belongs to the field of medical appliances, and particularly relates to a device for expanding a tube between two organs in an individual.

### Description of the Related Art

The following description is merely an introduction of some background knowledge and does not constitute any limitation to the invention.

At present, devices for expanding a tube between two organs in an individual such as a human body or a mammal are a necessary prerequisite for performing some surgeries. Especially when surgical treatment or drug introduction is required for pathological changes inside the organs, as the tube between the two organs is very small, it is expected to expand the tube to facilitate the surgical treatment. When the tube connected between the two organs is occluded, it is also required to expand the tube for treatment purposes.

For example, for middle-aged and elderly people, the bladder and the prostate are prone to pathological changes, especially prostatic hyperplasia, which is a common disease. At present, an expansion balloon arranged at a catheter is usually used to expand the urethra (the tube between the two organs) so as to realize the treatment of the patient.

However, there are still some defects in the above expansion. This requires improvement of the existing expansion device so as to expand the tube better and reduce trauma to the organs or the tube, and also allow the operator or the doctor to operate more conveniently.

### BRIEF SUMMARY OF THE INVENTION

In order to solve some problems in the traditional technology, the invention provides an expansion device, including: a catheter body having a guide portion, an expansion portion and an infusion portion. The expansion portion includes an expansion balloon, and the expansion balloon is configured to be capable of expanding a tube between two organs.

In some embodiments, the expansion portion includes an expansion balloon, which covers a surface of the expansion portion in a natural state, i.e., directly or indirectly covers an outer surface of the catheter at the expansion portion. When the balloon is full of gas or liquid, a volume of the balloon increases, so that the tube is expanded. The tube here includes a urethra, such as a urethra between the bladder and the prostate or a urethra in the genitals, and also includes a passageway between other two organs. In some embodiments, the balloon is distensible and is typically made of an elastic film. The balloon is naturally loose without expansion in an initial state. When the balloon is filled with gas or liquid under a certain pressure, the volume of the balloon increases, and the size of the balloon also increases, so that the size of the tube increases. For example, when the balloon is not inflated, the volume of the inner cavity is very small, and once inflated, the balloon expands, and the volume of the inner cavity increases.

In some embodiments, the device further includes a limiting element, and the limiting element allows the size of the expansion balloon to be relatively stable when the volume or size of the expansion balloon increases. "Stable" here is generally understood as that when the expansion balloon is filled with gas or liquid, its size increases as compared with in the natural state, for example, its volume, diameter or perimeter increases, and the limiting element functions to maintain the expansion balloon with the larger size within a stable size range, for example, maintain the size change or fluctuation within a certain range. That is, when the volume of the expansion balloon increases to a certain value, the limiting element limits the volume of the expansion balloon from further increasing or maintains the volume within a relatively stable range.

This is because the expansion balloon has such properties: the expansion balloon is made of an elastic material, and increasing the size of the expansion balloon is to increase the size of the tube. To increase the size of the tube, it is required to exert a certain pressure, which must be greater than the pressure of the gas or liquid itself, inside the expansion balloon, and an additional pressure is also required, for example, the inside of the expansion balloon is maintained above 1 atm, such as 1.5, 2, 2.5, 3 or 3.5 atm, for a period of time, such as 20 s, 1 min or 10 min. The pressure exerted and the time maintained depend on the specific conditions of the tube and the size of the tube to be expanded. If the expansion balloon is made of the elastic material, maintaining above the atmosphere requires continuously exerting a pressure, for example, continuously filling the expansion balloon with gas or liquid. However, as the pressure increases, the volume of the balloon made of the elastic material changes constantly. In this case, although the pressure for expanding the tube is exerted, it is impossible to control the required size of expansion of the tube, and as the pressure increases, it is impossible to determine the specific size of expansion of the tube. In addition, as the pressure increases, the balloon made of the elastic material may burst when possible. Once the balloon bursts, the air or liquid in the balloon leaks out, and the pressure decreases, which leads to unsuccessful expansion, causing surgical failures. In view of this technical defect, a control element is provided in the invention. The control element can maintain the expanded balloon at a stable size. In this case, even if a higher pressure is exerted in the balloon for a longer time, although the pressure of the balloon increases, the volume or size is kept relatively stable. In this way, an expansion pressure can be continuously exerted on the tube, and the size of expansion of the tube can be kept stable, so that the tube can be expanded to different sizes while the size is kept stable, thereby avoiding surgical failures or trauma to the expanded tube.

In some embodiments, the limiting element is located outside the expansion balloon, which can limit the expansion balloon to a stable size of expansion. In some embodiments, the limiting element may be a net, a film, an inelastic film or an inelastic net. In some embodiments, the limiting element may wrap the whole expansion balloon or part of the expansion balloon. Preferably, the limiting element wraps the whole expansion balloon. In some embodiments, in a natural state, the limiting element has a fixed size and does not deform. The expansion balloon has a natural state and a gas-filled or liquid-filled state. For example, the expansion balloon 11 in the natural state almost has no gas or liquid inside, and the inner surface contacts each other or directly covers the surface of the catheter. Once the expansion balloon is filled with gas or liquid or the size or volume of the expansion balloon increases after gas or liquid filling, it naturally expands. However, since the limiting element outside limits the size or volume, the expansion balloon can only expand to the volume or size limited by the limiting element. This can stably maintain the volume and size of the expansion balloon, so that the pressure of expansion exerted on the tube is kept constant (even if the pressure inside the expansion balloon increases), and the expansion balloon is kept within a safe range without bursting. The fixed size of the limiting element is the expected size or maximum size of expansion of the tube. For example, if the urethra is expected to be expanded to a diameter of 2 cm, then the fixed diameter of the limiting element is 2 cm, and the maximum expansion size of the expansion balloon inside is 2 cm, regardless of the pressure in the expansion balloon.

In some embodiments, the limiting element covers an outer surface of the expansion balloon. The limiting element is also in the form of a balloon, but this balloon is an inelastic balloon, and this balloon does not need to be filled with gas inside, nor have sealing performance. For example, the limiting balloon may be made of a net, a fiber material or an inelastic material, but it is flexible and inelastic. The elastic balloon here is generally air-tight or liquid-tight, and is made of a flexible and elastic material. When the elastic balloon is filled with gas or liquid, it undergoes elastic deformation, so that its volume or size increases. In some embodiments, the limiting element is flexible, but inelastic and not substantially elastic. The "covering" in the invention may be understood as wrapping or directly or indirectly contacting. For example, the limiting element wraps the expansion balloon therein, or the limiting element wraps the expansion balloon, or the limiting element partially covers or wraps part of the expansion balloon.

Of course, in some embodiments, the limiting element may also be elastic, but not necessarily flexible. For example, the limiting element is a net enclosure woven from metal wires outside the expansion balloon, and the net has a fixed size and worse elastic strain performance than the expansion balloon. That is, when the expansion balloon is filled with gas or liquid, its size of expansion is constant under a fixed pressure, but as the pressure increases, the size also increases until the expansion balloon bursts. The limiting element is elastic, and as the pressure increases, the size of the limiting element may also increase. However, when the pressure increases to a certain level, the limiting element will not substantially change in size with the increase of the pressure, but will be maintained at a relatively constant size. In other words, when the pressure inside the expansion balloon increases to a certain value, the expansion balloon bursts; and when the pressure inside the limiting balloon increases to a certain value, i.e., the maximum pressure value, it also bursts. That is, the maximum pressure value over which the limiting balloon bursts is greater than the maximum pressure value over which the expansion balloon bursts, which is also a solution of the invention. The pressure may be realized by gas filling or liquid filling.

In some embodiments, when the expansion balloon is full of gas or liquid, the expansion balloon is filled with the gas or liquid such that the expansion balloon expands and the volume of the expansion balloon increases, and the limiting element is capable of keeping the volume of the expansion balloon within a constant range. In some embodiments, the limiting element is a limiting balloon covering the outer surface of the expansion balloon. In some embodiments, under a same pressure, the size of expansion of the limiting balloon is less than a size of expansion of the expansion balloon. In some embodiments, the limiting balloon is made of an inelastic material. In some embodiments, the limiting balloon is made of an inelastic but flexible material. The limiting balloon made of the flexible material can cover the surface of the expansion balloon no matter the expansion balloon inside expands or not. Since the limiting balloon is flexible, when it contacts the tube, it does not cause mechanical trauma and can smoothly expand the tube. In some embodiments, the limiting balloon has a fixed size, for example, a fixed diameter of 1, 2, 3, 4, 5, 6, 7 or 8 cm, or a fixed volume of 50, 80, 100, 200, 300, 400 or 800 ml. The diameter represents the size of expansion of the expansion balloon inside, and may also represent the expected size of expansion of the tube. Therefore, in some embodiments, the limiting balloon wraps the expansion balloon therein.

In some embodiments, the expansion balloon is made of an elastic material, and in a case where there is no gas or liquid filling inside, the expansion balloon covers a surface of the catheter.

In some embodiments, the expansion portion of the catheter further includes a first positioning balloon and a second positioning balloon, and the first, second and expansion balloons are each independently in communication with the infusion portion. The first and second positioning balloons mainly have a positioning function, and when the expansion balloon expands the tube, their positions are fixed. In some embodiments, when in use, the first positioning balloon is located in one of the organs, the second positioning balloon is located in the other organ, and the part of the catheter between the two positioning balloons is located in the tube between the organs. In some embodiments, the first positioning balloon is close to the guide portion, and the second positioning balloon is away from the guide portion or close to the infusion portion. In some embodiments, when in use, the first positioning balloon and the second positioning balloon are respectively located in the two organs.

In some embodiments, there is an appropriate distance between the first positioning balloon and the second positioning balloon, and the appropriate distance is configured to be located in the tube between the two organs. In some embodiments, the appropriate distance is part of the catheter, and the part of the catheter is provided with the expansion balloon and the limiting element. In this way, the expansion balloon and the limiting element are also located on the surface of the catheter, and the expansion balloon is located inside the limiting element, such as the limiting balloon.

In some embodiments, the expansion device further includes a first positioning balloon and a second positioning balloon, and the expansion balloon wraps the first and second positioning balloons therein. That is, when the limiting element is a balloon, it limits the size of expansion of the expansion balloon, and when the first and second positioning balloons are located in the expansion balloon, the sizes of expansion of the first and second positioning balloons are also limited. In this way, all the balloons (the first and second positioning balloons and the expansion balloon) on the expansion portion of the expansion device have a fixed maximum size of expansion, which prevents the maximum sizes of expansion of the first positioning balloon and the second positioning balloon from being inconsistent due to operation errors of different operators.

In some embodiments, the infusion portion at a tail end of the catheter is provided with a plurality of independent passageways, which are each independently in communication with the first positioning balloon, the second positioning balloon and the expansion balloon respectively and are each independently used for gas filling or liquid filling to increase the volume of each balloon. In some embodiments, a pressure, such as a pressure of greater than one atmosphere, may be exerted on each balloon. In some embodiments, the expansion balloon is filled with gas or liquid through the passageway to make the internal pressure greater than one atmosphere, such as 1, 1.5, 2.5, 3 or 3.5 atm. The pressure is exerted by gas filling or liquid filling with a syringe in communication with a pressure gauge, which is a ready-made commercial tool that can be purchased, such as a disposable balloon expansion pressure pump with the trade name "fervid".

In some embodiments, the infusion portion has a first passageway, a second passageway and a third passageway. The first passageway is in communication with the first positioning balloon, the second passageway is in communication with the second positioning balloon, and the third passageway is in communication with the expansion balloon. The first positioning balloon, the second positioning balloon and the expansion balloon are independent of each other and are not in communication with each other.

In some embodiments, the infusion portion is further provided with a guide wire passageway through which a guide wire runs, and this passageway allows the catheter to enter the human body along the guide wire. The two organs are a bladder and a prostate. The tube is a urethra, which includes a urethra between the bladder and the prostate and a urethra in the genitals.

Therefore, a guide wire passageway is further arranged in the catheter. This passageway is configured to allow a guide wire to run through, so that the catheter body can enter the organs inside the individual from outside the individual through the guide wire. In some embodiments, an end of the guide portion is open, so that when the end enters the bladder, urine can be discharged through the guide wire passageway.

In some embodiments, the catheter body is further covered with a protective film. The protective film covers the surface of the limiting element so as to protect the limiting element. In some embodiments, the protective film is also elastic and clings to the limiting element. In some embodiments, the covering film is a flexible elastic film. In some embodiments, the protective film covers the surface of the catheter body and has a smooth surface such that the catheter can enter the organs inside the human body along the guide wire.

### Beneficial effects

According to the invention, the limiting element arranged outside the expansion balloon can effectively control the size of expansion of the expansion balloon. Especially when the pressure of the expansion balloon increases, the limiting element prevents the size of the expansion balloon from increasing infinitely, so that the size of the expansion balloon is kept stable despite the high pressure inside. In this way, the tube to be expanded can be expanded accurately, and trauma is reduced. In addition, this allows different devices in the same batch to have a stable size of expansion for different tubes, thereby maintaining the consistency of surgeries. In addition, for the doctor's operation, the controllability is increased, and the result is unique and stable.

### BRIEF DESCRIPTION OF THE DRAWINGS

Fig. 1 is a schematic structural view of an expansion device in a specific embodiment of the invention (not expanded);
Fig. 2 is a schematic structural (assembly) view of the expansion device in a specific embodiment of the invention;
Fig. 3 is a schematic structural view of the expansion device in a specific embodiment of the invention (with a first positioning balloon 31 expanded);
Fig. 4 is a schematic structural view of the expansion device in a specific embodiment of the invention (with a second positioning balloon 21 expanded);
Fig. 5 is a schematic structural view of the expansion device in a specific embodiment of the invention (with an expansion balloon 11 expanded);
Fig. 6 is a schematic structural (assembly) view of the expansion device in a specific embodiment of the invention, in which , each ballon indenpdentical is fluid or air communication with a passageways ;
Fig.7A is a schematic structural view of the expansion device when in use in a specific embodiment of the invention (with the first positioning balloon expanded);
Fig. 7B is a schematic structural view of the expansion device when in use in a specific embodiment of the invention (with the second positioning balloon expanded); and
Fig. 7C is a schematic structural view of the expansion device when in use in a specific embodiment of the invention (with the expansion balloon expanded).

### DETAILED DESCRIPTION OF THE INVENTION

The structures involved in the invention or the technical terms used are further explained below. Unless otherwise specified, they shall be understood and explained according to the general terms commonly used in the art.

### Catheter

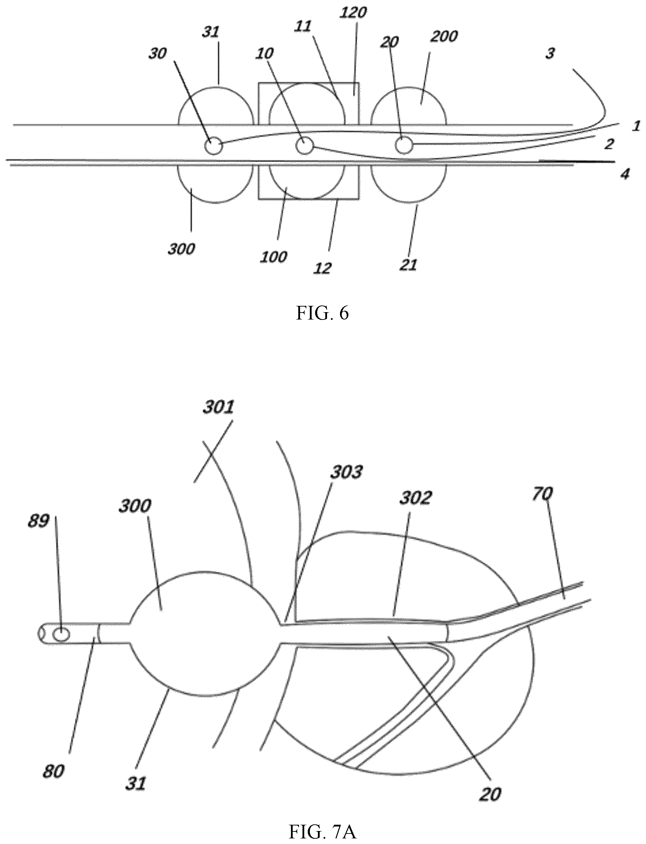

The invention provides a catheter, which is partially inserted into the body and partially located outside the body. The catheter includes an end for insertion into the body, and has an infusion portion outside the body. The end for insertion into the body is connected to the infusion portion through the catheter. The catheter here may actually be a flexible pipe, such as a plastic pipe, which has an opening 81 at the end 80 for insertion and an infusion portion 70 at the tail. The end with the opening 81 is configured to be inserted into the body, and the infusion portion 70 is in communication with the inside of the catheter through a plurality of passageways(1,2,3,4 in Fig.6). In some embodiments, as shown in Fig. 6, the infusion portion has an introduction passageway 4. A guide wire is inserted into the introduction passageway 4, so that the catheter can enter the body through the guide wire. After the catheter is introduced into the body, the guide wire may or may not be drawn out of the introduction passageway 4. Generally, the guide wire is inserted into the body through the opening outside the body. For example, when the catheter needs to enter the bladder through the urethra, the thin metal guide wire is allowed to enter the urethra of the genitals through the urine outlet of the genitals, pass through the prostate and then enter the bladder through the prostate. The introduction of the guide wire may be implemented with the aid of CT, B-scan or X-ray, which facilitates positioning and state monitoring. Then, the opening 81 at the end 80 of the catheter is allowed to enter the guide wire passageway of the catheter from the guide wire outside the body, then enter the urethra of the genitals along the guide wire, pass through the prostate and the urethra between the prostate and the bladder and enter the bladder. Since the bladder usually contains urine inside, once the urine flows out through the guide wire passageway 4 via the opening 81 or a hole 89, it indicates that the end for insertion has entered the bladder, and at this time, the liquid may be injected through the introduction passageway outside the body for cleaning.

### Expansion portion

In the invention, the catheter has an expansion portion 60. The expansion portion functions to expand the tube (urethra) between the bladder and the prostate to increase its size, in order to reduce the pressure of the urethra of the prostate and the pressure of the bladder. When the urethra becomes larger, the pressure is reduced, which facilitates urination and makes the urination smoother. Of course, the expansion portion may also expand the tube between another two organs in the body so as to make the liquid flow smoothly. As old people become older, the muscles degenerate, and the urethra becomes narrower, which may be one of the main causes of dysuria. Therefore, the device of the invention is especially suitable for old people to expand the the urethra as to make urethra becomes larger.

In order to facilitate the expansion of the tube, the catheter includes an expansion portion 60, which is generally located near an introduction end 80 of the catheter. When the catheter is inserted into the body, the expansion portion is expected to be located in the tube to be expanded or run through the tube, so that the tube can be expanded conveniently. In some embodiments, the expansion portion 60 is provided with an expansion balloon 11. The balloon 11 is made of an elastic material, such as nylon or Pebax. After being filled with gas or liquid, the balloon 11 expands, so that the balloon 11 expands from its natural state of no gas or liquid filling in the balloon, and becomes larger in volume or size, for example, in diameter. The larger size acts on the tube, so that it is expected to expand the tube through the expansion of the expansion balloon. Therefore, in some embodiments, the expansion balloon 11 covers an outer surface of the catheter at the expansion portion(cover the out surface of the plastic pipe on the expansion portion 60). After being filled with gas or liquid, the expansion balloon 11 radially expands around along the central axis of the catheter to form a cylinder or sphere with a larger diameter. The purpose of the increase in size is to increase the size of the tube against the contraction force of the muscles of the tube (like the urethra in odl man).

However, in actual use, since the balloon 11 is made of an elastic material (an elastic and flexible material), when gas or liquid is injected into the balloon 11, it is expected to keep the balloon 11 at a pressure higher than atmospheric pressure for a period of time, so that the balloon 11 has a large expanding force acting on the tube for a period of time. The pressure may be increased by continuously adding gas or liquid to the expansion balloon. However, as the pressure increases, the size of the expansion balloon will inevitably increase. When a high pressure is needed, the balloon may be unable to bear such a high pressure (the maximum pressure value over which the balloon bursts) due to its material so as to burst, which leads to unsuccessful expansion of the tube and the need for reoperation. Typically, a medical elastic material with a low price is preferred. Different elastic materials have different maximum pressures over which the balloon bursts. For example, when the elastic material is fixed and the size is fixed under natural conditions, in a case where the increased pressure of the gas or liquid inside that causes the balloon to burst due to expansion is fixed (the maximum pressure value of breaking or bursting), if the maximum pressure of bursting of some materials is 1.5 atm and the actual pressure needed to expand the tube is 2.5 atm, such materials cannot be used, because the balloon will burst and cause surgical failures in the pressue over than 1.5 atm. Thereby, there is a limit in selecting the material of the balloon. Only the materials with a pressure of bursting of higher than 2.5 atm can be selected as the material of the balloon. In addition, the pressure exerted by different operators on the inside of the expansion balloon 11 may vary or fluctuate. For example, if the specified pressure is 2.5 atm and the pressure actually exerted is 2 atm or above, such as 3 atm, the higher pressure will inevitably cause unnecessary trauma to the tube. For example, the increasing pressure expands the tube to a larger predermined size with the fix pressure 2.5 atm in the expansion balloon 11 , but the actuall pressure is high than 2.5 ( for example 3.0 atm) due to different doctor or nurse, which will inevitably cause trauma to the tube. Especially in some cases, it is expected to expand the tube to a desired size, which is relatively fixed, for example, from 0.5 mm to 1, 2, 3 or 4 cm. This size of expansion is expected to be stable and fixed, for example, at 1 cm, 2 cm or 4 cm. but, different doctor or nurse did not know if the pressure in the expansion balloon is stalbel.

In order to solve the above problem, the invention creatively provides a limiting element outside the expansion balloon, which can maintain the expansion balloon 11 at a constant volume when it expands to a certain volume. in this contidition, the internal pressure in the expansion balloon 11 can increase continuously, and it may increase to the expected pressure and even a pressure causing bursting due to expansion, so that the material of the expansion balloon can be selected from a wider range. In addition, since the volume of the expansion balloon is constant, the size is constant, and the size of expansion for the tube is also constant, which avoids errors caused by different operators. Sometimes the expansion of the tube is larger than the design, which causes unnecessary trauma.

In some embodiments, the limiting element includes a limiting balloon 12, and the limiting balloon wraps the expansion balloon 11 therein or the expansion balloon 11is located in the limiting balloon 12. In some embodiments, the limiting balloon 12 is made of a flexible but inelastic material, such as some nonwoven fabrics, cotton cloth, silk nets and ultrathin white nylon textiles. In some embodiments, the limiting balloon 12 has a fixed size in a natural state, for example, a fixed volume of 120 ml or a fixed diameter. In this way, even if the expansion balloon 11 inside is expanded by an exerted pressure, its maximum size of expansion is controlled by the limiting balloon 12, so that the size of expansion of the expansion balloon 11 is kept relatively constant. Even if the internal pressure in the expansion balloon 11 increases to a pressure causing bursting of the expansion balloon 11, the expansion balloon will not burst because of the protection of the limiting balloon 12 outside. The limiting balloon 12 may be made of any flexible but inelastic material, such as some metal net enclosures. The "balloon" in the limiting balloon may be air-permeable and liquid-permeable, such as gridded net enclosures, and may be made of a plastic material or a cloth, silk or fiber material made into a balloon shape. The expansion balloon 11 is arranged inside the limiting balloon 12. The "balloon" in the expansion balloon 11 and the first positioning balloon 31 and second positioning balloon 21 described later is generally made of a material impermeable to air or liquid, which may be a flexible but elastic material or an elastic material. In this way, the balloon covers the surface of the catheter at the expansion portion when in the natural state, and expands and increases in volume when being filled with gas or liquid, thereby realizing expansion or position limiting. Such materials include, for example, a nylon film, an elastic plastic film or any other elastic material, especially a medical elastic material suitable for entering the human body or an elastic material with high tightness.

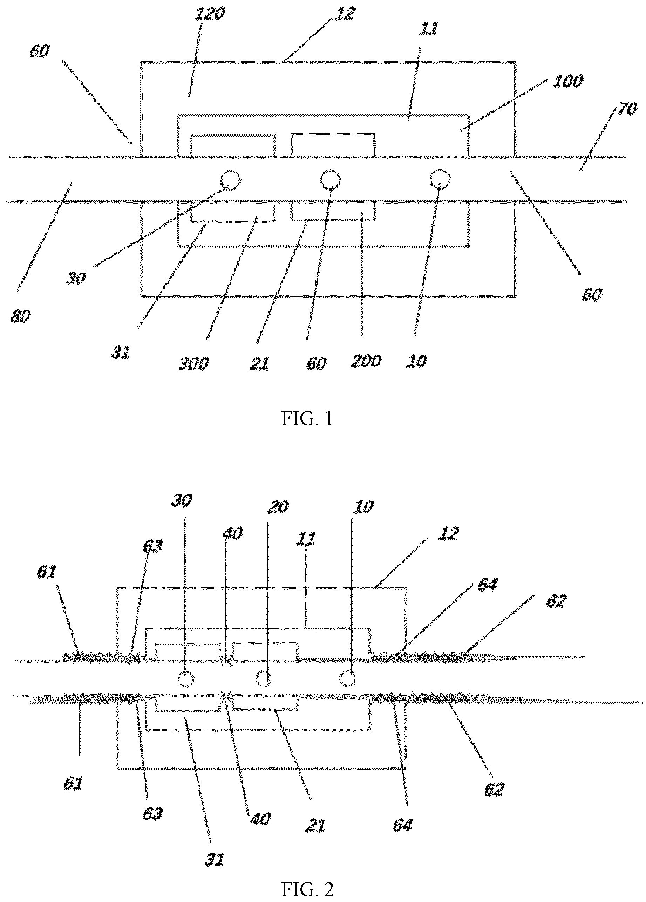

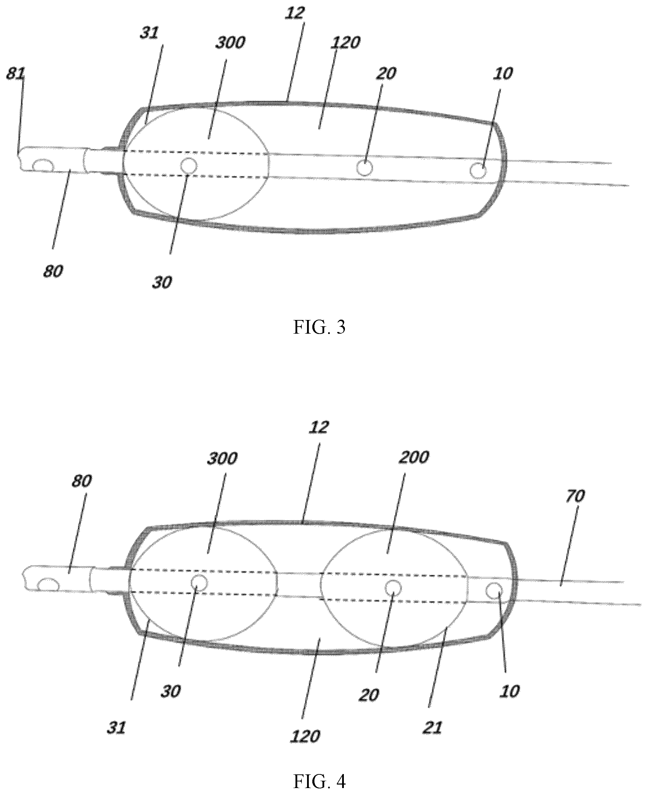

In some embodiments, the expansion device includes a first positioning balloon 31 and a second positioning balloon 21. The first positioning balloon 31 is close to the end for insertion of the catheter 80, and the second positioning balloon 21 is away from the end for insertion, but both of them(a first positioning balloon 31 and a second positioning balloon 21, the expansion balloon 11 and the limiting ballon 12 ) are located at the expansion portion 60. The expansion portion 60 on the catheter is close to the end for insertion 80. In some embodiments, the first positioning balloon 31 and the second positioning balloon 21 are located in the expansion balloon 11, and the expansion balloon 11 is located in the limiting balloon 12. For example, as shown in Fig. 1 to Fig. 5, the expansion balloon 11 is located at the expansion portion 60, and the expansion balloon 11 includes the first positioning balloon 31 and the second positioning balloon 21 therein. When the first and second positioning balloons are filled with liquid or gas, an expanded first balloon cavity 300 and an expanded second balloon cavity 200 are formed. When the expansion balloon 11 is filled with liquid or gas, an expanded expansion balloon cavity 100 is formed. For example, in the initial state, the first positioning balloon 31 and the second positioning balloon 21 cover the perimeter of the catheter or the outer surface of the catheter, the expansion balloon 11 covers the surfaces of the first and second positioning balloons, and the limiting balloon 12 covers the outer surface of the expansion balloon 11. The first, second and expansion balloons each have a passageway each independently in communication with the infusion portion, which is used for adding gas or liquid to the first, second or expansion balloon. Therefore, in some embodiments, the first positioning balloon 31 has a first through hole 30 therein, the second positioning balloon 21 has a second hole 20 therein, the expansion balloon 11 has a third through hole 10 therein, and the third through hole 10 is provided outside the first and second positioning balloons. All these through holes are provided in the wall of the catheter, and are each independently located in the balloon and each independently in communication with the passageway in the infusion portion(FIG. 6) .

The first positioning balloon 31 expands to form the first positioning balloon cavity 300, the second positioning balloon 21 expands to form the second positioning balloon cavity 200, and the expansion balloon 11 expands to form the expansion balloon cavity 100. The limiting balloon may be flexible but inelastic, and the volume of the limiting balloon cavity 120 formed by the limiting balloon is constant or substantially constant.

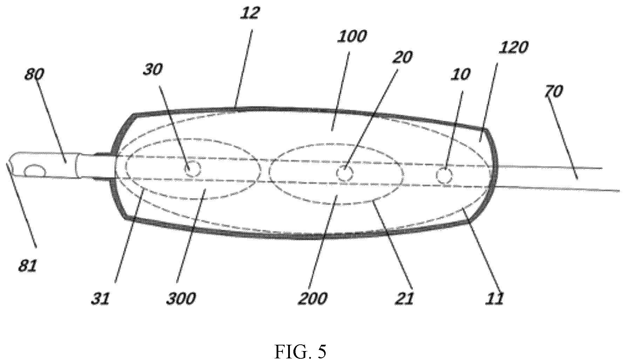

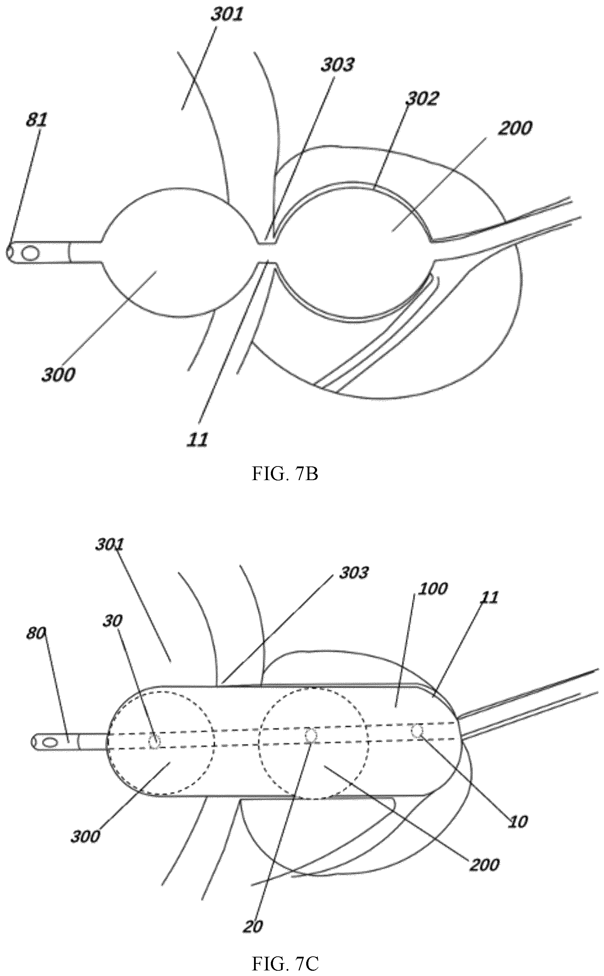

For example, Fig. 5 shows the expansion balloon 11, the first positioning balloon 31 and the second positioning balloon 21 in an expanded state after being filled with liquid. Typically, when the catheter is inserted into the bladder 301 (Fig. 7A), first, liquid is injected into the first expansion balloon 31 through the passageway 3 to make it expand and form an expanded positioning balloon cavity 300. Next, the first positioning balloon 31 is gently drawn outward close to the outlet of the bladder. Then, liquid is injected into the second positioning balloon 21 through the passageway 1 to make it expand and form an expanded balloon cavity 200. At this time, the second positioning balloon is located in the prostate 302, and the second positioning balloon is in a fixed position where the expansion portion of the catheter is locked. During the expansion, the position of the catheter in the tube is kept fixed due to the positioning of the first and second positioning balloons. In this case, the outer surface of the expansion balloon 11 indirectly contacts the tube 303 (the wall of the urethra) through the surface of the limiting balloon 12. At this time, the liquid is further injected into the expansion balloon 11 through the passageway 2 to increase its internal pressure and make it expand, such that the internal pressure of the balloon cavity 100 inside the expansion balloon 11 gradually increases to 2.5 atm or above. At this time, due to the presence of the limiting balloon 12 outside the expansion balloon 11, the maximum size or volume of expansion of the expansion balloon 11 is limited by the limiting balloon 11 in a stable size, so that its size is kept constant and the internal pressure can be effectively increased, thereby preventing the balloon cavity 100 inside the expansion balloon 11 from continuing expanding and bursting. The tube 303 can be effectively expanded by increasing the pressure for a period of time. Therefore, the limiting balloon 12 does not expand, but limits the size of expansion of the expansion balloon 11 stabllely. In this way, the size of expansion of the tube can be kept consistent with the size of the limiting balloon 12, for example, if the diameter of the limiting balloon is 2 cm, then the final size of expansion of the tube is 2 cm.

In addition, in this embodiment, since the first positioning balloon 31 and the second positioning balloon 21 are actually also located inside the limiting balloon 12 (as shown in Fig. 5), when the liquid is injected into the first and second positioning balloons, the maximum size of expansion of the first and second positioning balloons is also controlled by the imiting balloon 12 . When the limiting balloon 12 is a uniform cylinder, its maximum diameter is fixed. In this case, the maximum diameter of the first and second positioning balloons is limited by the limiting balloon 12, so that the sizes of expansion of the first positioning balloon, the second positioning balloon and the expansion balloon are actually all limited. This has the advantages as follows. First, although the first and second positioning balloons cannot expand the tube, a positioning function is needed. When the liquid is injected into the first positioning balloon 31, if the first positioning balloon reaches its maximum size (controlled by the limiting balloon 12), it indicates that the first positioning balloon has filled with the liquid. Moreover, even if a higher pressure is exerted subsequently, the first positioning balloon will not burst, thereby ensuring the success of the experiment. Similarly, the maximum size of expansion of the second positioning balloon 21 (located in the prostate) is also limited by the limiting balloon 12, which can reduce the trauma to the prostate. Different maximum sizes of expansion may be set for different people, and the diameter or volume of expansion of the first positioning balloon 31 and the second positioning balloon 21 in each product of one batch is kept consistent, thereby avoiding different volumes of expansion due to different operators. Without the presence of the limiting balloon 12, the maximum size of expansion of the first positioning balloon 31 and the second positioning balloon 21 is not the same for everyone and not be esaly controlled, and if the diameter is too big for someone, it may squeeze or even hurt the glands. Second, since the first positioning balloon 31, the second positioning balloon 21 and the expansion balloon 11 are all limited by the limiting balloon 12, in the final expansion step, they form an overall shape limited by the limiting balloon that can expand the tube to a constant size.

In some embodiments, for example, as shown in Fig. 6, the expansion balloon 11 is located between the first positioning balloon 31 and the second positioning balloon 21. The expansion balloon 11 located therebetween is actually located inside the tube and functions to expand the tube, and the first positioning balloon 31 and the second positioning balloon 21 located at two sides function to fix the position of the expansion balloon 11 and prevent the expansion balloon from moving during the expansion, so that the tube can be effectively expanded at a fixed position. In this case, simply providing the limiting balloon 12 outside the expansion balloon 11 can also obtain the effects of the invention.

In some embodiments, in order to distinguish the positions and operation sequence of the first positioning balloon 31, the second positioning balloon 21 and the expansion balloon 11, the passageway 3 connected to the first positioning balloon 31 is the shortest, the passageway 2 connected to the expansion balloon 11 is the longest, and the length of the passageway 1 connected to the second positioning balloon 21 is in the middle, which indicate the operation sequence. The liquid is first injected into the first positioning balloon 31 through the shortest passageway 3 (through the through hole 30) to make it expand, then the liquid is injected into the second positioning balloon 21 (through hole20) through the passageway 1 with the length in the middle, and finally, the liquid is injected into the expansion balloon 11 through the passageway 2(through hole 10) to make it expand. The passageway 4 is configured to allow the guide wire to run through. All the passageways are each independently in communication with the corresponding through hole. For example, the passageway 3 is in communication with the through hole 30, the passageway 2 is in communication with the through hole 10, and the passageway 1 is in communication with the through hole 20 (as shown in the figure 6). The outlets of these passageways are each in communication with the corresponding through hole in the balloon, and the inlets are located at the infusion portion 70 or extend outward.

### Tube

The tube of the invention is a passageway whose width needs to be increased through the expansion balloon, such as a urethra, which may be, for example, a urethra between the bladder and the prostate or a urethra in the genitals, and certainly may also be other tubes. These tubes are formed by muscle tissue and are contractive. When the tube needs to be expanded, it is required to overcome the contraction force to increase its size. For example, if the size of the tube is originally 0.5 mm and the tube needs to be expanded to 1, 2 or 3 cm, while the liquid is injected into the expansion balloon, the pressure needs to be increased, and the pressure acts on the inner wall of the tube so as to expand the tube. The pressure here may be 1.5, 2, 2.5, 3 or 3.5 atm.

### Manufacturing process

The invention may provide a manufacturing process. First, a catheter is provided, such as a pipe. The catheter has openings at both ends. The opening 81 ,89 at the end for insertion is in communication with the inside of the bladder, and an infusion portion is at the other end. The infusion portion is provided with 4 passageways. The passageway 4 is configured to allow a guide wire to run through the whole catheter such that the catheter can enter into the body, for example, enter the bladder through the prostate. A wall of the catheter at the end for insertion near the expansion portion 60 is sequentially provided with three holes 30, 20, 10 from near to far. Next, a flexible first elastic sleeve is provided and sleeved on an outer wall of the catheter through the end for insertion. Then, in the middle of the catheter 40, i.e., at the catheter 40 between the first hole 30 and the second hole 20, a fixing member is used to divide the flexible catheter into two sections which are air-tight or liquid-tight with each other, so that the elastic sleeve tightly clings to the surface of the catheter between the first hole 30 and the second hole 20. Moreover, with the same method, two ends 63, 64 of the sleeve are allowed to tightly cling to the catheter to form a seal so as to form a first positioning balloon 31 and a second positioning balloon 21 which are each independently in communication with the first hole 30 and the second hole 20. The first hole 30 is in communication with the third passageway 3 of the infusion portion, and the second hole 20 is in communication with the second passageway 1 of the infusion portion. Then, a second elastic sleeve is provided, which has a larger length than the first elastic sleeve. The second elastic sleeve is sleeved on the expansion portion 60 of the catheter from the end for insertion. The first positioning balloon 31 and the second positioning balloon 21 are allowed to be located in the second sleeve, and the third hole 10 located outside the second and first positioning balloons is allowed to be located in the second elastic sleeve. This second elastic sleeve is sealed at two ends 61, 62 and tightly clings to the wall of the catheter, thereby forming an expansion balloon 11. At this time, a limiting balloon 12 (which is flexible but inelastic) is also provided. The limiting balloon is also in the form of a sleeve, and its inner cavity has a fixed diameter of about 1 to 2 cm or a fixed volume of 100 to 200 ml. The expansion portion 60 is allowed to run through the limiting balloon, and the limiting balloon is sealed at two ends, the positions of which may be the same as those of the two ends 61, 62 of the expansion balloon, so that the expansion balloon is located in the limiting balloon 12. Thereby, the expansion balloon 11 can be located in the limiting balloon 12, and the first and second positioning balloons 31, 21 can be located in the expansion balloon 11, as shown in Fig. 2. Of course, a similar method may be used to manufacture the structure shown in Fig. 6.

In a preferred embodiment, a flexible elastic protective film is sleeved outside the limiting balloon 12. The protective film has a smooth surface, which functions to protect the limiting balloon 12 from damage (when the surface of the limiting balloon is a rough fabric or net cover) or protect the tube from trauma due to direct contact, and also functions as lubrication to facilitate the insertion of the catheter into the body. On the other hand, when the expansion balloon 11, the first positioning balloon 31 and the second positioning balloon 21 are expanded, the protective film also needs to be expanded. Of course, the maximum size of expansion is controlled by the limiting balloon 12 inside (as shown in Fig. 5). The protective film may cover the expansion portion, and may also cover the surface of the whole catheter between the end for insertion and the infusion portion.

The following describes another embodiment of the invention:
An expansion device includes: a catheter, configured to be capable of insertion into two organs in a human body which are in communication with each other through a tube, and having a guide portion, an expansion portion and an infusion portion. The expansion portion includes an expansion balloon, and the expansion balloon is configured to be capable of expanding the tube. The expansion device further includes a limiting element, and the limiting element is capable of limiting or controlling a size of expansion of the expansion balloon to keep the expansion balloon within a limited size.

In some embodiments, the limiting element is located outside the expansion balloon. In some embodiments, the limiting element covers an outer surface of the expansion balloon. In some embodiments, when the expansion balloon is full of gas or liquid, the expansion balloon is filled with the gas or the liquid such that the expansion balloon expands and a volume of the expansion balloon increases, and the limiting element is capable of keeping the volume of the expansion balloon within a constant range. In some embodiments, the limiting element is a limiting balloon, and the expansion balloon is located in the limiting balloon. In some embodiments, under a same pressure, a size of expansion of the limiting balloon is less than the size of expansion of the expansion balloon. In some embodiments, the limiting balloon is made of an inelastic material. In some embodiments, the limiting balloon has a fixed size. In some embodiments, the limiting balloon is made of a flexible inelastic material. In some embodiments, the expansion balloon is made of an elastic material, and in a case where there is no gas or liquid filling inside, the expansion balloon covers a surface of the catheter. In some embodiments, the expansion balloon further includes a first positioning balloon and a second positioning balloon therein, and the first, second and expansion balloons are each independently in communication with the infusion portion. In some embodiments, the first positioning balloon is close to the guide portion, and the second positioning balloon is away from the guide portion. In some embodiments, when in use, the first positioning balloon and the second positioning balloon are respectively located in the two organs. In some embodiments, there is an appropriate distance between the first positioning balloon and the second positioning balloon, and the appropriate distance is configured to be located in the tube between the two organs. In some embodiments, the expansion device further includes a first positioning balloon and a second positioning balloon, and the expansion balloon is located between the first positioning balloon and the second positioning balloon. In some embodiments, the first positioning balloon, the second positioning balloon and the expansion balloon are each independently in communication with the infusion portion. In some embodiments, the infusion portion has a first passageway, a second passageway and a third passageway. The first passageway is in communication with the first positioning balloon, the second passageway is in communication with the second positioning balloon, and the third passageway is in communication with the expansion balloon. In some embodiments, the two organs are a bladder and a prostate, and the tube is a urethra. In some embodiments, the expansion balloon, the first positioning balloon and the second positioning balloon are made of an elastic film, and after being filled with gas or liquid, the balloons expand and their volumes increase. In some embodiments, the catheter body is further covered with a protective film. The protective film covers the surface of the limiting element. The protective film is also an elastic film. In some embodiments, the maximum pressure value over which the limiting balloon bursts is greater than the maximum pressure value over which the expansion balloon bursts.

All patents and publications mentioned in the specification of the invention indicate that these are disclosed techniques in the art and can be used by the invention. All patents and publications cited herein are likewise listed in the references as if each publication is specifically and separately referenced. The invention described herein may be implemented in the absence of any one or more elements, and one or more limitations, which are not specifically stated herein. For example, the terms "comprising", "essentially consisting of" and "consisting of" in each example here may be replaced by the rest 2 terms. The term "a/an" here merely means "one", but does not exclude including 2 or more instead of including only one. The terms and expressions which have been employed here are descriptive rather than restrictive, and there is no intention to suggest that these terms and expressions in this description exclude any equivalents, but it is to be understood that any appropriate changes or modifications can be made within the scope of the invention and appended claims. It can be understood that the embodiments described in the invention are some preferred embodiments and features. Any person of ordinary skill in the art can make some modifications and changes according to the spirit of the description of the invention. These modifications and changes are also considered to fall within the scope of the invention and the scope limited by independent claims and dependent claims.

## Claims

1. An expansion device, comprising:
a catheter, configured to be capable of insertion into two organs in a human body which are in communication with each other through a tube, comprising a guide portion, an expansion portion and an infusion portion; wherein the expansion portion comprises an expansion balloon, and the expansion balloon is configured to be capable of expanding the tube, wherein the expansion device further comprises a limiting element, and the limiting element is capable of limiting or controlling a size of expansion of the expansion balloon to keep the expansion balloon within a limited size.

2. The device according to claim 1, wherein the limiting element is located outside the expansion balloon.

3. The device according to claim 1, wherein the limiting element covers an outer surface of the expansion balloon.

4. The device according to claim 3, wherein when the expansion balloon is full of gas or liquid, the expansion balloon is filled with the gas or the liquid such that the expansion balloon expands and a volume of the expansion balloon increases, and the limiting element is capable of keeping the volume of the expansion balloon within a constant range.

5. The device according to any of claims 1 to 4, wherein the limiting element is a limiting balloon, and the expansion balloon is located in the limiting balloon.

6. The device according to claim 5, wherein under a same pressure, a size of expansion of the limiting balloon is less than the size of expansion of the expansion balloon.

7. The device according to claim 5, wherein the limiting balloon is made of an inelastic material.

8. The device according to claim 5, wherein the limiting balloon has a fixed size.

9. The device according to claim 5, wherein the limiting balloon is made of a flexible inelastic material.

10. The device according to any of claims 1 to 9, wherein the expansion balloon is made of an elastic material, and in a case where there is no gas or liquid filling inside, the expansion balloon covers a surface of the catheter.

11. The device according to any of claims 1 to 10, wherein the expansion balloon further comprises a first positioning balloon and a second positioning balloon therein, and the first, second and expansion balloons are each independently in communication with the infusion portion.

12. The device according to claim 11, wherein the first positioning balloon is close to the guide portion, and the second positioning balloon is away from the guide portion.

13. The device according to claim 12, wherein when in use, the first positioning balloon and the second positioning balloon are respectively located in the two organs.

14. The device according to claim 13, wherein there is an appropriate distance between the first positioning balloon and the second positioning balloon, and the appropriate distance is configured to be located in the tube between the two organs.

15. The device according to any of claims 1 to 10, wherein the expansion device further comprises a first positioning balloon and a second positioning balloon, and the expansion balloon is located between the first positioning balloon and the second positioning balloon.
